## Europäisches Patentamt

## European Patent Office

## Office européen des brevets

(11) Veröffentlichungsnummer: **0 164 500**
**B1**

(12) # EUROPÄISCHE PATENTSCHRIFT

(45) Veröffentlichungstag der Patentschrift:
**18.10.89**

(51) Int. Cl.⁴: **A 61 M 16/00**

(21) Anmeldenummer: **85102704.5**

(22) Anmeldetag: **09.03.85**

(54) **Steuerbare Ventileinheit.**

(30) Priorität: **14.06.84 DE 3422066**

(43) Veröffentlichungstag der Anmeldung:
**18.12.85 Patentblatt 85/51**

(45) Bekanntmachung des Hinweises auf die Patenterteilung:
**18.10.89 Patentblatt 89/42**

(84) Benannte Vertragsstaaten:
**AT DE FR GB NL SE**

(56) Entgegenhaltungen:
**EP-A- 0 051 503**
**EP-A- 0 107 590**
**EP-A- 0 121 255**
**DE-A- 2 947 659**
**DE-B- 2 424 025**
**US-A- 4 127 121**

(73) Patentinhaber: **Drägerwerk Aktiengesellschaft,
Moislinger Allee 53-55, D-2400 Lübeck 1 (DE)**

(72) Erfinder: **Baum, Marcel, Dipl.-Ing., Lorbeergasse 15/7,
A-1030 Wien (AT)**

## Beschreibung

Die Erfindung betrifft eine steuerbare Ventileinheit für ein Beatmungssystem nach dem Oberbegriff des Anspruchs 1.

Aus der DE-PS 917 210 ist eine Vorrichtung zur künstlichen Beatmung bekannt, bei der ein Beatmungsanschluss über druckabhängig gesteuerte zwangsweise gekoppelte Ventile in einem offenen System abwechselnd mit der Ansaugseite und mit der Ausblaseseite eines Injektors verbunden wird.

Die DE-PS 946 258 beschreibt ein Atmungsgerät mit einem Inspirations- und einem Exspirationszweig, die jeweils mit einem Druckgebläse und mit einem Sauggebläse verbunden sind. Durch eine in beiden Zweigen wirksame Kulissenschiebersteuerung werden positive und negative Beatmungsdrücke von entsprechender Dauer erzeugt. Es handelt sich ebenfalls um ein offenes System, welches den Nachteil eines hohen Verbrauches an Atemgas aufweist.

Derartige Systeme sind wegen der Steuerträgheit ihrer mechanischen Bauteile nicht verwendbar, wenn eine relativ hohe Impulsfolgefrequenz der Beatmungsimpulse bei hoher Flankensteilheit erreicht werden soll.

In der DE-AS 2 424 025 ist ein Beatmungsgerät mit Inspirationszweig und Exspirationszweig beschrieben, welche Teile einer geschlossenen Ringleitung bilden. Im Inspirationszweig ist ein Einatemventil und im Exspirationszweig ein Ausatemventil vorhanden. Zur Druckerzeugung dient ein in seiner Drehrichtung umsteuerbares Gebläse, welches wechselseitig mit seiner Druckseite an den Inspirationszweig und nach Drehrichtungsumkehr mit seiner Saugseite an den Exspirationszweig angeschlossen wird. Eine solche Anordnung erfordert eine komplizierte Drehrichtungsumsteuerung des Gebläses und ist nur für relativ langsame Impulsfolgefrequenzen verwendbar.

Gasförderelemente in Ringleitungen sind bei Narkosegeräten unter anderem durch die US-PS 4 127 121 bekannt. Dort dient jedoch das Gasförderelement ausschliesslich zur Umwälzung einer Spülgasströmung und erzeugt keine in einem Beatmungssystem erforderlichen Inspirations- bzw. Exspirationsimpulse.

In der EP-A 107 590 sind pneumatisch betriebene Membranventile, die die Merkmale nach dem Oberbegriff des Anspruchs 1 aufweisen, genannt, welche jedoch keine Vorrichtungen zur Erzeugung von Unter- und Überdrücken aufweisen.

Die Erfindung geht von der Aufgabe aus, eine Ventilanordnung zu schaffen, die in ihrer Schalteigenschaft so präzise und kurzzeitig betätigbar ist, dass sie die schnell aufeinanderfolgenden Inspirations- und Exspirationspulse einer schnellen Beatmungsfrequenz (oberhalb 200 Inspirationspulse/min) ohne merkliche Beeinflussung der Impulsform zu steuern in der Lage ist.

Die Lösung der Aufgabe erfolgt mit den kennzeichnenden Merkmalen des Anspruchs 1.

In der Inspirationsphase erzeugt das Gasförderelement, welches zweckmässig als Mitteldruckgebläse ausgebildet sein kann, in Förderrichtung einen Überdruck, der bei geöffnetem Einatemventil und geschlossenem Ausatemventil über den Inspirationszweig das Atemgas in die Lunge des Beatmeten drückt. Umgekehrt wird bei geschlossenem Einatemventil und geöffnetem Ausatemventil durch den an der Saugseite des Gasförderelementes anstehenden Unterdruck über den Exspirationszweig die Lunge entleert. Die Dauer der Inspirations- und Exspirationsphase bzw. die Höhe der erzeugten Unter- bzw. Überdruckwerte lassen sich vorteilhaft durch eine geeignete Einstellung der Förderleistung sowie der Impulsbreite für die Inspirations- und Exspirationsimpulse einstellen. Eine steuerbare Ventileinheit, welche in einem Beatmungssystem, gegebenenfalls aber auch davon unabhängig, eingesetzt werden kann, ist zweckmässig so aufgebaut, dass das Einatemventil und das Ausatemventil als Membranventile mit pneumatischem Antrieb ausgebildet sind, wobei die Membran einen Steuerhilfsraum von einem Gasführungsraum trennt und bei der der Steuerhilfsraum Mittel zur Überdruck- und Unterdruckerzeugung enthält. Bei einer zweckmässigen Ausführungsform werden in einer solchen steuerbaren Ventileinheit kombinierte Injektor- und Ejektorpaare verwendet. Ihre Düsen lassen sich durch die Steuervorrichtung mit einer Druckluftquelle verbinden, wobei das Steuergerät vorzugsweise entsprechend angeordnete Magnetventile ansteuert. In einem solchen Aufbau lassen sich Inspirations- bzw. Exspirationsimpulse hoher Flankensteilheit und hoher Impulsfolgefrequenz erzeugen.

Die steuerbare Ventileinheit kann mit ihrem Einatem- und Ausatemventil im Inspirationszweig und Exspirationszweig jedes bekannten Beatmungsgerätes verwendet werden, sie eignet sich jedoch besonders für den Einsatz in dem angegebenen Beatmungssystem der eingangs beschriebenen Art, wenn hohe Impulsfolgefrequenzen oberhalb der natürlichen Beatmungsfrequenz und hohe Flankensteilheit der Impulse erzeugt werden sollen.

In der Zeichnung ist ein Ausführungsbeispiel des Gegenstandes der Erfindung schematisch dargestellt; es zeigen:

Fig. 1 ein Schaltungsdiagramm des Beatmungssystems,

Fig. 2 eine schematische Darstellung der steuerbaren Ventileinheit mit den zugeordneten Schaltelementen.

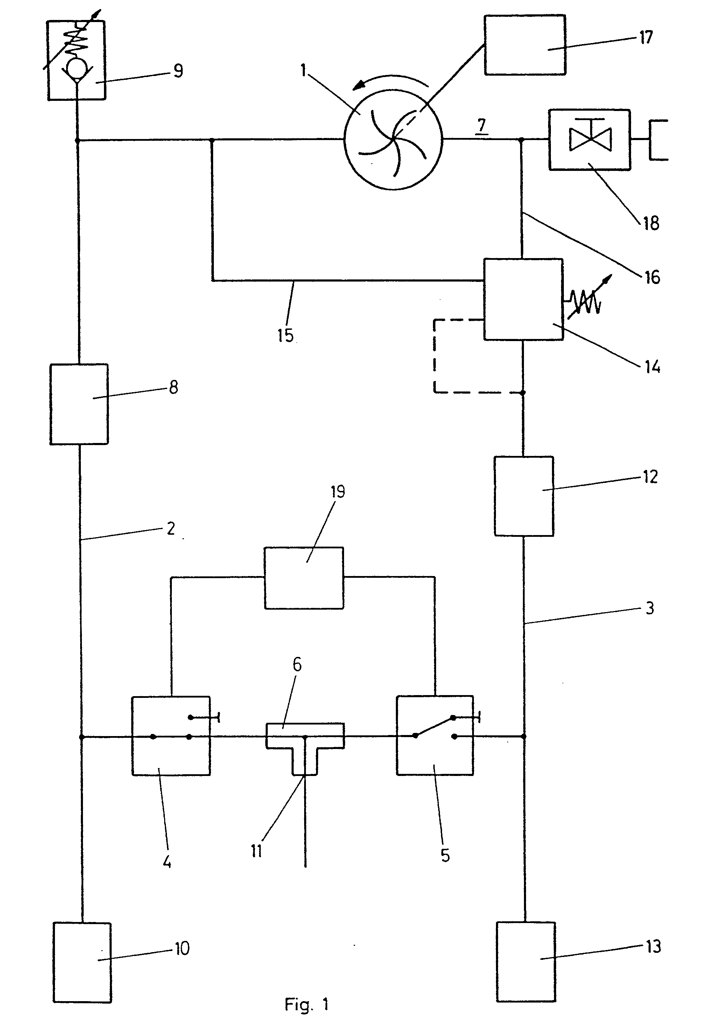

In Fig. 1 ist ein Gasförderelement als Mitteldruckgebläse 1 angeordnet, welches zusammen mit einem Inspirationszweig 2, einem Exspirationszweig 3 sowie einem Einatemventil 4, einem Ausatemventil 5 und einem T-Stück 6 zu einer Ringleitung 7 verbunden ist. Die Drehrichtung des Gebläses 1 und damit die Gasförderrichtung ist durch einen Pfeil angezeigt. Der Inspirationszweig 2 beginnt am Auslass des Gebläses 1 und endet in der Abzweigung am T-Stück 6. Der Exspirationszweig 3 führt von der Abzweigung des T-Stücks 6 bis zur Ansaugseite des Gebläses 1. Im Inspirationszweig 2 befindet sich in Strömungsrichtung hinter dem Gebläse 1 und vor einem Anfeuchter 8 ein einstellbares Überdruckventil 9. Hinter dem Anfeuchter 8 und vor dem Einatemventil 4 ist ein Druckspeicher 10 mit dem Inspirationszweig 2 verbunden. Das T-Stück 6 verbindet Inspirations- und Exspirationszweig 2, 3 und ermöglicht mit seinem Ansatz 11 den Anschluss der beatmeten Person, der in bekannter Weise über eine Atemmaske bzw. über einen Trachealtubus erfolgen kann.

Am Anfang des Exspirationszweiges 3 befindet sich das Ausatemventil 5, dem ein $CO_2$-Absorber 12 nachgeschaltet ist. Zwischen dem $CO_2$-Absorber 12 und dem Ausatemventil 5 ist ein Unterdruckspeicher 13 an den Exspirationszweig 3 angeschlossen. Hinter dem $CO_2$-Absorber 12 liegt ein Unterdruckregler 14, welcher so angeordnet ist, dass über eine Bypass-Leitung 15 und eine Leitung 16 die Druckseite hinter dem Gebläse 1 mit der Saugseite vor diesem Gebläse überbrückt ist. Vor der Ansaugseite des Gebläses 1 ist ausserdem ein Frischgasförderelement 18 zur dosierten Einspeisung von Atemgas in die Ringleitung 7 an den Exspirationszweig 3 angeschlossen.

Zur Steuerung des Einatemventils 4 und des Ausatemventils 5 dient ein in bekannter Weise mit elektronischen Bauelementen aufgebautes Steuergerät 19.

Das in Fig. 1 dargestellte Beatmungssystem ermöglicht durch den in der Zeichnung nicht näher erläuterten regelbaren Drehantrieb 17 des Gebläses 1 eine entsprechende Anpassung an die Druckwerte und Zeitdauer der Inspirations- bzw. Exspirationsimpulse. Der durch das Gebläse 1 im Exspirationszweig 3 erzeugte Unterdruck ist durch den Unterdruckregler 14 einstellbar. Die Folgefrequenz und die Zeitdauer von Inspirationsphase und Exspirationsphase werden durch die wechselseitige Ansteuerung des Einatemventils 4 und des Ausatemventils 5 durch das Steuergerät 19 bestimmt. Das Überdruckventil 9 ermöglicht die Einstellung der Atemmittellage.

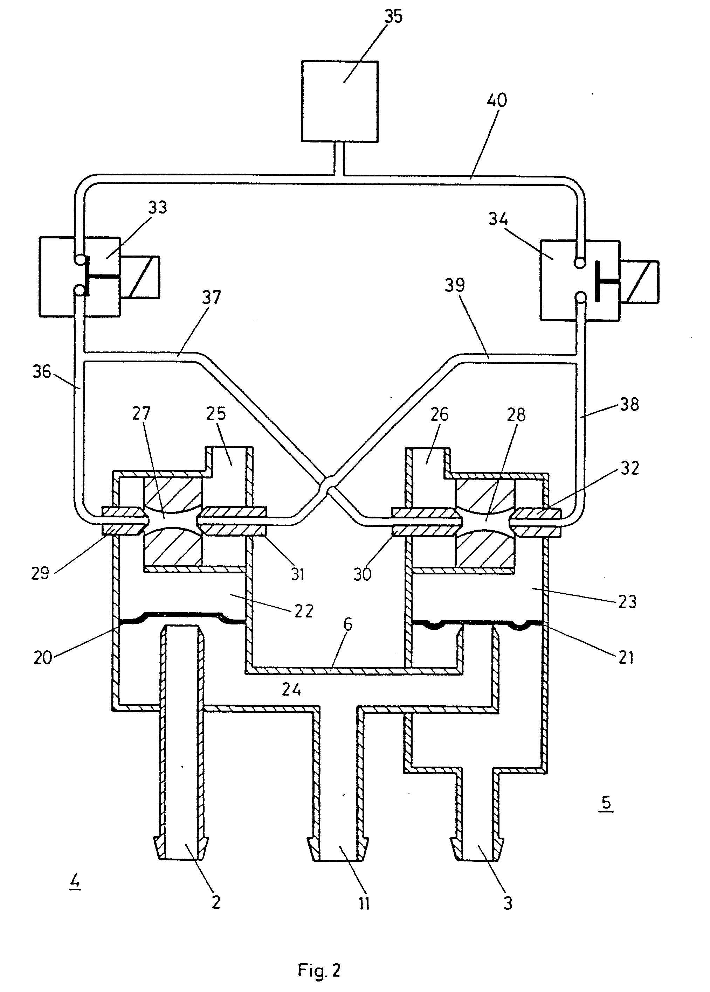

In Fig. 2 ist das Einatemventil 4 geöffnet und das Ausatemventil 5 geschlossen dargestellt. Beide Ventile 4, 5 sind Membranventile, wobei die Membranen 20, 21 jeweils einen Steuerhilfsraum 22, 23 von dem durch den verbleibenden Inspirationszweig 2 und den verbleibenden Exspirationszweig 3 gebildeten Gasführungsraum 24 abtrennen.

In den Steuerhilfsräumen 22, 23, welche jeweils über Auslassstutzen 25, 26 in die Umgebungsatmosphäre münden, sind kombinierte Injektor- und Ejektoreinheiten 27, 28 angeordnet, welche jeweils eine Ejektordüse 29, 32 und eine Injektordüse 30, 31 aufweisen. Diese Düsen sind über Verbindungsleitungen 36, 37, 38, 39 mit Magnetventilen 33, 34 verbunden. Die Druckluftquelle 35, die einen Überdruck von etwa 2-5 bar erzeugt, ist an die Magnetventile 33, 34 mit einer verzweigten Anschlussleitung 40 anzuschliessen.

In der gezeigten Einatmungsphase mit geöffnetem Einatemventil 4 und geschlossenem Ausatemventil 5 wird in dem Steuerhilfsraum 22 ein Unterdruck und in dem Steuerhilfsraum 23 ein Überdruck erzeugt. Hierzu ist die Druckluftquelle 35 durch das geschlossene Magnetventil 33 mit der Ejektordüse 29 und mit der Injektordüse 30 der kombinierten Injektor-Ejektoreinheiten 27, 28 verbunden. Durch die Ejektordüse 29 wird Luft aus dem Steuerhilfsraum 22 abgepumpt und durch den Auslassstutzen 25 in den Umgebungsraum geführt. Dabei entsteht ein Unterdruck, welcher die Membran 20 öffnet und dadurch den Inspirationszweig 2 mit dem Ansatz 11 verbindet.

Die Druckluftzufuhr zur Injektordüse 30 bewirkt das Ansaugen von Umgebungsluft aus dem Auslassstutzen 26 in den Steuerhilfsraum 23, so dass ein entsprechender Überdruck aufgebaut wird, welcher die Membran 21 schliesst und damit den Exspirationszweig 3 absperrt. In diesem Betriebszustand werden die Ejektordüse 32 und die Injektordüse 31 durch das geöffnete Magnetventil 34 drucklos gehalten.

Nach Ablauf der im Steuergerät 19 vorgegebenen Dauer des Einatmungsimpulses wird das Magnetventil 34 geschlossen und das Magnetventil 33 geöffnet. Damit erfolgt unter Umkehrung der beschriebenen Vorgänge die Einleitung der Exspirationsphase durch Öffnung des Ausatemventils 5, wobei gleichzeitig das Einatemventil 4 in die Schliessstellung übergeht.

Eine derartige steuerbare Ventileinheit, wie sie im Vorangehenden unter Bezug auf Fig. 2 beschrieben wurde, arbeitet besonders trägheitsarm und kann daher die bei der Hochfrequenzbeatmung gewünschten hohen Impulsfolgefrequenzen mit hoher Flankensteilheit der Einzelimpulse realisieren.

## Patentansprüche

1. Steuerbare Ventileinheit für ein Beatmungsgerät, welche ein Einatemventil (4) und ein Ausatemventil (5) enthält, wobei das Einatemventil (4) und das Ausatemventil (5) als Membranventil (20, 21) mit pneumatischem Antrieb ausgebildet sind, wobei jeweils die Membran (20, 21) einen Steuerhilfsraum (22, 23) von einem Gasführungsraum (24) trennt, dadurch gekennzeichnet, dass jeder Steuerhilfsraum (22, 23) ein kombiniertes Injektor- und Ejektorpaar (27, 28) aufweist, dass der Einlass der Injektor- und Ejektor-Düsen (29, 30, 31, 32) durch Ventile (33, 34) mit einer Druckluftquelle (35) verbindbar sind, und dass der Auslass der Injektordüsen in den Steuerhilfsraum (22, 23) und der Auslass der Ejektordüsen über einen Auslassstutzen (25, 26) in den Umgebungsraum münden.

2. Steuerbare Ventileinheit nach Anspruch 1, dadurch gekennzeichnet, dass zur Steuerung der Düsen (29, 30, 31, 32) zwei vom Steuergerät (19) angesteuerte Magnetventile (33, 34) vorgesehen sind, welche die Druckluftquelle (35) beim Einatemvorgang mit dem Ejektorteil des Einatemventils (4) und mit dem Injektorteil des Ausatemventils (5) und beim Ausatemvorgang mit dem Injektorteil des Einatemventils (4) und dem Ejektorteil des Ausatemventils (5) verbinden.

## Claims

1. A controllable valve unit for a respirator, comprising an inhalation valve (4) and an exhalation valve (5), the inhalation valve (4) and the exhalation valve (5) being designed as pneumatically driven diaphragm valves (20, 21), each diaphragm (20, 21) separating an auxiliary control chamber (22, 23) from a gas supply chamber (24), characterised in that each auxiliary control chamber (22, 23) comprises a combined injector and ejector pair (27, 28), in that the inlets of the injector and ejector nozzles (29, 30, 31, 32) are connected via valves (33, 34) with a pressure gas source (35), and in that the outlet of the injector nozzle opens into the auxiliary control chamber (22, 23) and the outlet of the ejector nozzle opens via an outlet socket (25, 26) into the surrounding space.

2. A controllable valve unit according to claim 1, characterised in that in order to control the nozzles (29, 30, 31, 32), two magnetic valves (33, 34) are provided which are actuated by the control device (19) and which during the inhalation procedure connect the pressure gas source with the ejector part of the inhalation valve (4) and with the injector part of the exhalation valve (5) and during the exhalation process connect the pressure gas source with the injector part of the inhalation valve (4) and the ejector part of the exhalation valve (5).

## Revendications

1. Unité commandée à soupapes pour appareil de respiration artificielle, comprenant une soupape d'inspiration (4) et une soupape d'expiration (5), la soupape d'inspiration (4) et la soupape d'expiration (5) étant conformées en soupapes à membrane (20, 21) avec commande pneumatique, chaque membrane (20,21) séparant une chambre auxiliaire de commande (22, 23) d'une chambre d'amenée de gaz (24), caractérisée en ce que chaque chambre auxiliaire de commande (22, 23) présente un couple combiné injecteur et éjecteur (27, 28), en ce que les entrées des buses formant injecteur et éjecteur (29, 30, 31, 32) sont raccordables par l'intermédiaire de soupapes (33, 34) avec une source d'air comprimé (35), et en ce que les sorties des buses formant injecteurs débouchent dans la chambre auxiliaire de commande (22, 23) et les sorties des buses formant éjecteurs débouchent, par l'intermédiaire d'une tubulure de sortie (25, 26), dans l'espace ambiant.

2. Unité commandée à soupapes selon la revendication 1, caractérisée en ce que sont prévues, pour la commande des buses (29, 30, 31, 32), deux soupapes magnétiques (33, 34) pilotées par l'appareil de contrôle (19), qui lors du processus d'inspiration relient la source d'air comprimé (35) avec la partie éjecteur de la soupape d'inspiration (4) et avec la partie injecteur de la soupape d'expiration (5), et qui lors du processus d'expiration relient cette source avec la partie injecteur de la soupape d'inspiration (4) et avec la partie éjecteur de la soupape d'expiration (5).

Fig. 1

Fig. 2